# EUROPEAN PATENT APPLICATION

(11) **EP 4 411 742 A1**
(43) Date of publication of application: **07.08.2024**
(21) Application number: 22875580.7
(22) Date of filing: 28.07.2022
(51) Int. Cl.: G16B 40/00

(54) **MEASURABLE AND PREFERABLE FEATURE SELECTION METHOD, PROGRAM FOR SELECTING MEASURABLE AND PREFERABLE FEATURE, AND MEASURABLE AND PREFERABLE FEATURE SELECTION DEVICE**

(30) Priority: 29.09.2021 JP 2021159737
(71) Applicant: FUJIFILM Corporation, Tokyo 106-8620 (JP)
(72) Inventor: NAGASE, Masaya, Ashigarakami-gun, Kanagawa 258-8538 (JP)
(74) Representative: Hughes, Andrea Michelle
(86) International application number: PCT/JP2022/029058
(87) International publication number: WO 2023/053703

(57) **Abstract**

Provided are a measurable suitable feature amount selection method, a measurable suitable feature amount selection program, and a measurable suitable feature amount selection device that select a measurable feature amount. A measurable suitable feature amount selection method includes a data set input step of inputting a data set (32), a feature amount candidate extraction step of extracting a feature amount candidate (34) from the data set (32), a feature amount selection candidate extraction step of extracting a feature amount selection candidate (36), a measurement element design step of extracting a feature amount, for which the design of a measurement element has succeeded, as a measurable suitable feature amount (38), and a measurement element design result notification step of feeding back a result of whether the design of the measurement element has succeeded or failed in the measurement element design step to at least one of the feature amount candidates (34), the feature amount selection candidate extraction step, or the measurement element design step. A measurable suitable feature amount, for which the measurement element is capable of being designed and which predicts or discriminates features of a sample, is selected from the feature amount candidates.

## Description

### BACKGROUND OF THE INVENTION

### 1. Field of the Invention

The present invention relates to a measurable suitable feature amount selection method, a measurable suitable feature amount selection program, and a measurable suitable feature amount selection device, and particularly to a measurable suitable feature amount selection method, a measurable suitable feature amount selection program, and a measurable suitable feature amount selection device that can measure a selected feature amount.

### 2. Description of the Related Art

A problem of predicting or discriminating features of a given sample from a given feature amount is a central issue handled by modern machine learning and the like. For example, in the field of gene analysis, there are a multi-class cancer classification problem and the like based on a deoxyribonucleic acid (DNA) methylation pattern.

Among these, there is a problem called feature amount selection in which there are a large number of feature amount candidates and a small number of feature amounts are selected in advance from them. However, in practice, it is sufficient to simply select the feature amount. In some cases, it is separately required to design measurement means (measurement element) for the selected feature amount.

For example, in the gene analysis problem, a marker gene required for features of a sample, such as cancer, is selected on the basis of a data set in which a large number of genes (feature amount) have been measured by a microarray, a sequencer, or the like, and a polymerase chain reaction (PCR) primer (measurement element) is designed for the selected marker gene. As a method for designing the primer, a procedure of selecting a feature amount and designing a measurement element for the feature amount is usually performed.

For example, Emese Meglecz et al., "a user-friendly program to select microsatellite markers and design primers from large sequencing projects", Bioinformatics, Volume 26, issue 3, 1 February 2010, Pages 403-404 discloses a method for collectively performing selection of a marker from sequence data and primer design. Further, JP2019-528729A discloses a method that derives a predictive lead from a primer for a feature amount and determines a feature amount set.

### SUMMARY OF THE INVENTION

The method disclosed in Emese Meglecz et al., "a user-friendly program to select microsatellite markers and design primers from large sequencing projects", Bioinformatics, Volume 26, issue 3, 1 February 2010, Pages 403-404 selects an appropriate marker from sequence data, designs a primer for the appropriate marker, and presents the primer to the user. In the method, the possibility that the design of the primer for the selected marker will fail is not considered. The method disclosed in JP2019-528729A is limited to improving efficiency or being used for a plurality of targets, and JP2019-528729A does not disclose any primer design. Further, specific means for reflecting the predictive lead in the determination of a feature amount set has not been described. In the method disclosed JP2019-528729A, the possibility that the design of the primer will fail is not considered.

The design of the primer is not always successful. In a case where the success rate is low, the feature amount needs to be selected again. In addition, in a case where the combination effect of the feature amounts is large and it is not possible to design the measurement element for the selected feature amount, it is not possible to implement a combination of the selected feature amounts and to achieve the expected prediction and discrimination performance.

The present invention has been made in view of these circumstances, and an object of the present invention is to provide a measurable suitable feature amount selection method, a measurable suitable feature amount selection program, and a measurable suitable feature amount selection device that, in a case where feature amount selection and measurement element design are required, select a measurable feature amount by cooperation therebetween.

In order to achieve the object of the present invention, according to the present invention, there is provided a measurable suitable feature amount selection method for selecting a feature amount that is used to predict or discriminate features of a sample and is measurable. The measurable suitable feature amount selection method comprises: a data set input step of inputting a data set of a sample group in which values of a plurality of the feature amounts are recorded; a feature amount candidate extraction step of extracting a feature amount candidate from the data set; a feature amount selection candidate extraction step of selecting a feature amount for predicting or discriminating the features of the sample and extracting a feature amount selection candidate; a measurement element design step of designing a measurement element that is capable of measuring the feature amount and extracting a feature amount, for which the design of the measurement element has succeeded, as a measurable feature amount; and a measurement element design result notification step of feeding back a result of whether the design of the measurement element has succeeded or failed in the measurement element design step to the feature amount selection candidate extraction step or notifying the feature amount selection candidate extraction step of the measurable feature amount. The feature amount selection candidate extraction step is performed on the feature amount candidate or the measurable feature amount, and the measurement element design step is performed on the feature amount candidate or the feature amount selection candidate. In the feature amount selection candidate extraction step or the measurement element design step, a measurable suitable feature amount, for which the measurement element is capable of being designed and which predicts or discriminates the features of the sample, is selected from the feature amount candidates.

According to an aspect of the present invention, preferably, in the feature amount selection candidate extraction step, one or a plurality of the feature amounts are selected from the feature amount candidates, and the feature amount selection candidate is extracted. Preferably, in the measurement element design step, the measurement element is designed for the feature amount selection candidate. Preferably, in the measurement element design result notification step, at least one of the following is performed: in a case where it is determined that the design of the measurement element has failed on the basis of the result of the measurement element design step, the feature amount determined as a failure is fed back to the feature amount candidates and is deleted from the feature amount candidates; in a case where it is determined that the design of the measurement element has succeeded on the basis of the result of the measurement element design step, the feature amount determined as a success is used as the measurable suitable feature amount, the measurable suitable feature amount is fed back to the feature amount selection candidate extraction step, and conditions of the feature amount selection candidate extraction step are controlled; or the measurable suitable feature amount is fed back to the measurement element design step, and conditions of the measurement element design step are controlled. Preferably, the feature amount selection candidate extraction step and the measurement element design step are repeated until a desired number of the measurable suitable feature amounts are capable of being selected.

According to an aspect of the present invention, preferably, the measurement element design step is performed on the feature amount candidates, and a feature amount for which the design of the measurement element is determined to succeed is used as a measurable feature amount candidate. Preferably, in the measurement element design result notification step, a notification of the measurable feature amount candidate is sent to the feature amount selection candidate extraction step. Preferably, in the feature amount selection candidate extraction step, the extraction is performed until a desired number of the measurable suitable feature amounts are capable of being selected from the measurable feature amount candidates.

According to an aspect of the present invention, preferably, the feature amount selection candidate extraction step includes a first feature amount selection candidate extraction step of extracting the feature amount selection candidate with first feature amount selection means having a weak constraint and a second feature amount selection candidate extraction step of extracting the feature amount selection candidate with second feature amount selection means having a strong constraint. Preferably, the first feature amount selection candidate extraction step is performed on the feature amount candidates to extract first feature amount selection candidates. Preferably, the measurement element design step is performed on the first feature amount selection candidates, and a feature amount for which the design of the measurement element is determined to succeed is used as a measurable feature amount selection candidate. Preferably, in the measurement element design result notification step, a notification of the measurable feature amount selection candidate is sent to the second feature amount selection candidate extraction step. Preferably, in the second feature amount selection candidate extraction step, the extraction is performed until a desired number of the measurable suitable feature amounts are capable of being selected from the measurable feature amount selection candidates.

According to an aspect of the present invention, preferably, the measurement element design step includes a first measurement element design step of designing the measurement element for the feature amount with first measurement element design means having a weak constraint and a second measurement element design step of designing the measurement element for the feature amount with second measurement element design means having a strong constraint. Preferably, the first measurement element design step is performed on the feature amount candidates, and a feature amount for which the design of the measurement element is determined to succeed is used as a measurable feature amount candidate. Preferably, in the measurement element design result notification step, a notification of the measurable feature amount candidate is sent to the feature amount selection candidate extraction step, and the feature amount selection candidate extraction step is performed on the measurable feature amount candidates to extract a measurable feature amount selection candidate. Preferably, the second measurement element design step is performed on the measurable feature amount selection candidates, a feature amount for which the design of the measurement element is determined to succeed is extracted as the measurable suitable feature amount, and the extraction is performed until a desired number of the measurable suitable feature amounts are capable of being selected.

According to an aspect of the present invention, preferably, in the feature amount selection candidate extraction step, a feature amount selection candidate is selected from the feature amount candidates or the measurable feature amounts by one feature amount selection means selected from a plurality of feature amount selection means. Preferably, the measurement element design step is performed on the feature amount candidates or the feature amount selection candidates by one measurement element design means selected from a plurality of measurement element design means, and a feature amount for which the design of the measurement element is determined to succeed is selected as a measurable feature amount candidate. Preferably, in the measurement element design result notification step, a notification of the measurable feature amount candidate is sent to the feature amount selection candidate extraction step. Preferably, the measurable suitable feature amount selection method further comprises a repetition step of optionally repeating the feature amount selection candidate extraction step and the measurement element design step, and the measurable suitable feature amount for which the measurement element is capable of being designed is selected in stages by the repetition step.

According to an aspect of the present disclosure, preferably, the feature amount selection candidate extraction step is performed in ascending order of constraint in the repetition step, and the measurement element design step is performed in ascending order of constraint in the repetition step.

According to an aspect of the present invention, preferably, the feature amount is information of a gene, and the measurement element is gene measurement means.

According to an aspect of the present invention, preferably, the information of the gene is information of a DNA methylation site, and the gene measurement means is a primer.

According to an aspect of the present invention, preferably, a problem of predicting or discriminating the features of the sample is a multi-class classification problem of determining which of N classes the sample belongs to.

In order to achieve the object of the present invention, a measurable suitable feature amount selection program according to the present invention causes a computer to execute the above-described measurable suitable feature amount selection method.

In order to achieve the object of the present invention, according to the present invention, there is provided a measurable suitable feature amount selection device for selecting a feature amount that is used to predict or discriminate features of a sample and is measurable. The measurable suitable feature amount selection device comprises a processor. The processor is configured to have: a data set input process of inputting a data set of a sample group in which values of a plurality of the feature amounts are recorded; a feature amount candidate extraction process of extracting a feature amount candidate from the data set; a feature amount selection candidate extraction process of selecting a feature amount for predicting or discriminating the features of the sample and extracting a feature amount selection candidate; a measurement element design process of designing a measurement element that is capable of measuring the feature amount and extracting a feature amount, for which the design of the measurement element has succeeded, as a measurable feature amount; and a measurement element design result notification process of feeding back a result of whether the design of the measurement element has succeeded or failed in the measurement element design process to the feature amount selection candidate extraction process or notifying the feature amount selection candidate extraction process of the measurable feature amount. The feature amount selection candidate extraction process is performed on the feature amount candidate or the measurable feature amount, and the measurement element design process is performed on the feature amount candidate or the feature amount selection candidate. In the feature amount selection candidate extraction process or the measurement element design process, a measurable suitable feature amount, for which the measurement element is capable of being designed and which predicts or discriminates the features of the sample, is selected from the feature amount candidates.

According to an aspect of the present invention, preferably, in the feature amount selection candidate extraction process, one or a plurality of the feature amounts are selected from the feature amount candidates, and the feature amount selection candidate is extracted. Preferably, in the measurement element design process, the measurement element is designed for the feature amount selection candidate. Preferably, in the measurement element design result notification process, at least one of the following is performed: in a case where it is determined that the design of the measurement element has failed on the basis of the result of the measurement element design process, the feature amount determined as a failure is fed back to the feature amount candidates and is deleted from the feature amount candidates; in a case where it is determined that the design of the measurement element has succeeded on the basis of the result of the measurement element design process, the feature amount determined as a success is used as the measurable suitable feature amount, the measurable suitable feature amount is fed back to the feature amount selection candidate extraction process, and conditions of the feature amount selection candidate extraction process are controlled; or the measurable suitable feature amount is fed back to the measurement element design process, and conditions of the measurement element design process are controlled. Preferably, the feature amount selection candidate extraction process and the measurement element design process are repeated until a desired number of the measurable suitable feature amounts are capable of being selected.

According to an aspect of the present invention, preferably, the measurement element design process is performed on the feature amount candidates, and a feature amount for which the design of the measurement element is determined to succeed is used as a measurable feature amount candidate. Preferably, in the measurement element design result notification process, a notification of the measurable feature amount candidate is sent to the feature amount selection candidate extraction process. Preferably, in the feature amount selection candidate extraction process, the extraction is performed until a desired number of the measurable suitable feature amounts are capable of being selected from the measurable feature amount candidates.

According to the present invention, in a case where feature amount selection and measurement element design are required, it is possible to select a measurable suitable feature amount which is a measurable feature amount.

### BRIEF DESCRIPTION OF THE DRAWINGS

Fig. 1 is a block diagram illustrating a configuration of a measurable suitable feature amount selection device.
Fig. 2 is a block diagram illustrating a configuration of a processing unit.
Fig. 3 is a flowchart illustrating a measurable suitable feature amount selection method according to a first embodiment.
Fig. 4 is a flowchart illustrating a measurable feature amount selection method according to the related art.
Fig. 5 is a flowchart illustrating a measurable suitable feature amount selection method according to a second embodiment.
Fig. 6 is a flowchart illustrating a measurable suitable feature amount selection method according to a third embodiment.
Fig. 7 is a flowchart illustrating a measurable suitable feature amount selection method according to a fourth embodiment.
Fig. 8 is a flowchart illustrating a measurable suitable feature amount selection method according to a fifth embodiment.
Fig. 9 is a graph illustrating results of examples.

### DESCRIPTION OF THE PREFERRED EMBODIMENTS

Hereinafter, a measurable suitable feature amount selection method, a measurable suitable feature amount selection program, and a measurable suitable feature amount selection device according to the present invention will be described with reference to the accompanying drawings.

### <Schematic Configuration of Measurable Suitable Feature Amount Selection Device>

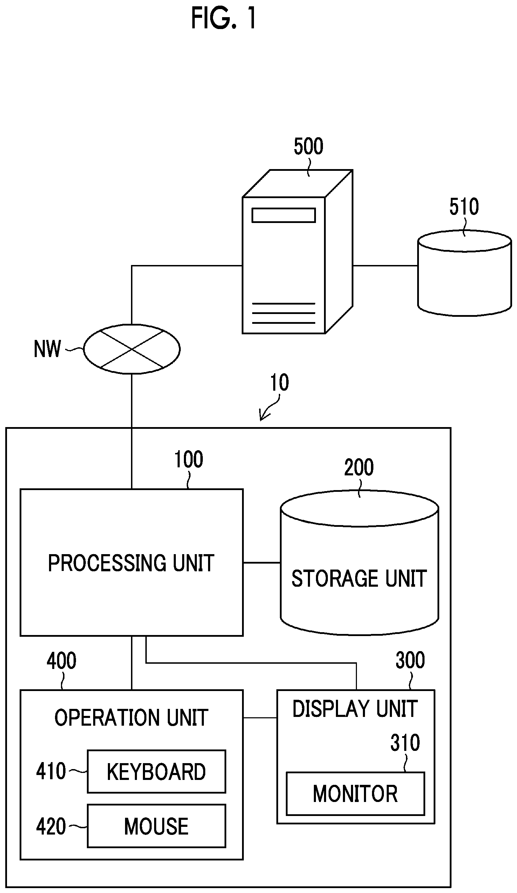

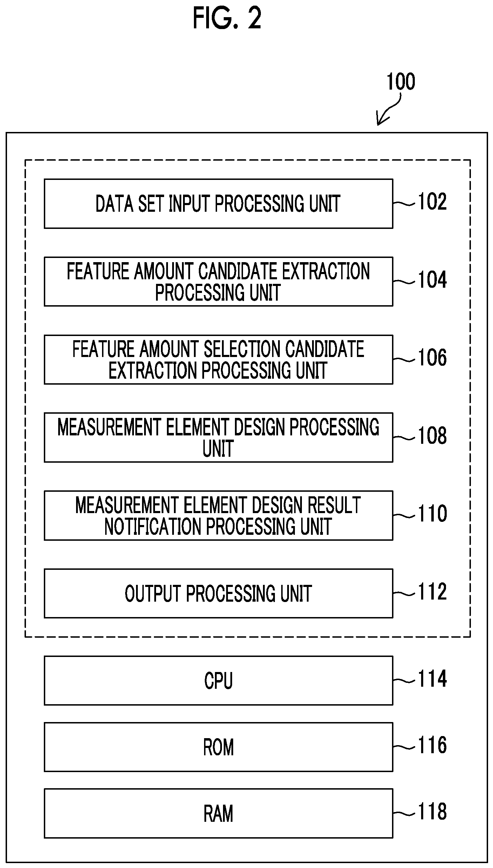

Fig. 1 is a diagram illustrating a schematic configuration of the measurable suitable feature amount selection device. As illustrated in Fig. 1, a measurable suitable feature amount selection device 10 comprises a processing unit 100 (a processor or a computer), a storage unit 200, a display unit 300, and an operation unit 400 which are connected to one another such that they transmit and receive necessary information. These components can be installed in various forms. These components may be installed in one place (in one housing, one room, or the like) or may be installed in places separated from each other and connected via a network. In addition, the measurable suitable feature amount selection device 10 (a data set input processing unit 102; see Fig. 2) is connected to an external server 500 and an external database 510 via a network NW, such as the Internet, and can acquire information, such as a sample for selecting a measurable suitable feature amount and a learning data set, as needed.

### <<Configuration of Processing Unit>>

As illustrated in Fig. 2, the processing unit 100 comprises the data set input processing unit 102, a feature amount candidate extraction processing unit 104, a feature amount selection candidate extraction processing unit 106, a measurement element design processing unit 108, a measurement element design result notification processing unit 110, an output processing unit 112, a central processing unit (CPU) 114, a read only memory (ROM) 116, and a random access memory 118 (RAM). The data set input processing unit 102 performs an input process of inputting a data set of a sample group, in which values of a plurality of feature amounts have been recorded, from the storage unit 200 or a storage device on the network. The feature amount candidate extraction processing unit 104 performs an extraction process of extracting feature amount candidates from the input data set. The feature amount selection candidate extraction processing unit 106 performs an extraction process of selecting a feature amount for predicting or discriminating features of a sample and extracting feature amount selection candidates. The measurement element design processing unit 108 performs an extraction process of designing a measurement element that can measure a feature amount and extracting the feature amount, for which the design of the measurement element has succeeded, as a measurable feature amount. The measurement element design result notification processing unit 110 performs a notification process of feeding back a result of whether the design of the measurement element by the measurement element design processing unit 108 has succeeded or failed to the feature amount selection candidate extraction processing unit 106 or notifying the feature amount selection candidate extraction processing unit 106 of the measurable feature amount. The feature amount selection candidate extraction processing unit 106 processes the feature amount candidate processed and extracted by the feature amount candidate extraction processing unit 104 or processes the measurable feature amount which has been processed by the measurement element design processing unit 108 and for which the design of the measurement element is determined to be possible. Further, the measurement element design processing unit 108 processes the feature amount candidate processed and extracted by the feature amount candidate extraction processing unit 104 or processes the feature amount selection candidate processed and extracted by the feature amount selection candidate extraction processing unit 106. The measurement element design result notification processing unit 110 feeds back the success or failure of the design of the measurement element to the feature amount selection candidate extraction processing unit 106 or notifies the feature amount selection candidate extraction processing unit 106 of the measurable feature amount, and each processing is performed to select a measurable suitable feature amount, for which the measurement element can be design and which predicts or discriminates the features of the sample, from the feature amount candidates. The output processing unit 112 outputs processing conditions or processing results by displaying, storing, printing, or the like. In addition, the processes by each of these units is performed under the control of the CPU 114 (a processor and a computer). Further, in the present invention, the feature amount selected by feature amount selection is referred to as a feature amount selection candidate, and the feature amount for which the measurement element can be designed is referred to as a measurable feature amount. Furthermore, the feature amount for which the measurement element can be designed and which predicts or discriminates the features of the sample is referred to as a measurable suitable feature amount.

The functions of each unit of the processing unit 100 can be implemented by various processors and recording media. The various processors include, for example, a central processing unit (CPU) which is a general-purpose processor that executes software (program) to implement various functions. In addition, the various processors also include a graphics processing unit (GPU) which is a processor specialized for image processing, and a programmable logic device (PLD) which is a processor whose circuit configuration can be changed after manufacturing, such as a field programmable gate array (FPGA). In a case where learning or recognition of the image is performed, the configuration using the GPU is effective. Further, the various processors also include a dedicated electric circuit which is a processor having a dedicated circuit configuration designed to execute a specific process such as an application specific integrated circuit (ASIC).

The functions of each unit may be implemented by one processor or may be implemented by a plurality of processors of the same type or different types (for example, a plurality of FPGAs, a combination of the CPU and the FPGA, or a combination of the CPU and the GPU). In addition, a plurality of functions may be implemented by one processor. A first example of the configuration in which a plurality of functions are configured by one processor is an aspect in which one processor is configured by a combination of one or more CPUs and software and implements a plurality of functions. A representative example of this aspect is a computer. A second example of the configuration is an aspect in which a processor that implements the functions of the entire system using one integrated circuit (IC) chip is used. A representative example of this aspect is a system on chip (SoC). As described above, various functions are configured using one or more of the above-described various processors as a hardware structure. In addition, specifically, an electric circuit (circuitry) obtained by combining circuit elements, such as semiconductor elements, can be used as the hardware structure of the various processors. The electric circuit may be an electric circuit that implements the above-described functions using a logical sum, a logical product, a logical negation, an exclusive logical sum, and a logical operation of a combination thereof.

In a case where the processor or the electric circuit executes software (program), codes that can be read by a computer (for example, various processors or electric circuits constituting the processing unit 100 and/or a combination thereof) in the executed software are stored in a non-transitory recording medium, such as the ROM 116, and the computer refers to the software. The software stored in the non-transitory recording medium includes a program (measurable suitable feature amount selection program) for executing the measurable suitable feature amount selection method according to the present invention and data (data related to the acquisition of learning data, data used to select the measurable suitable feature amount, and the like) used in a case of execution. The codes may be recorded on non-transitory recording media, such as various magneto-optical recording devices and semiconductor memories, instead of the ROM 116. In a case of processes using software, for example, the RAM 118 is used as a transitory storage area. In addition, data stored in an electronically erasable and programmable read only memory (EEPROM) (not illustrated) can also be referred to. The storage unit 200 may also be used as the "non-transitory recording medium".

Details of the process by the processing unit 100 having the above-described configuration will be described below.

### <<Configuration of Storage Unit>>

The storage unit 200 is configured by various storage devices, such as a hard disk and a semiconductor memory, and a control unit therefor and can store the sample for selecting the measurable suitable feature amount, the learning data set, feature amount selection means for executing the feature amount selection candidate extraction process, execution conditions and results thereof, measurement element design means for executing a measurement design process, execution conditions and results thereof, the selected measurable suitable feature amount, and the like.

### <<Configuration of Display Unit>>

The display unit 300 comprises a monitor 310 (display device) that is configured by a display, such as a liquid crystal display, and displays input information, information stored in the storage unit 200, the results of the process by the processing unit 100, and the like. The monitor 310 may be configured by a touch panel display and may receive an instruction input by a user.

### <<Configuration of Operation Unit>>

The operation unit 400 comprises a keyboard 410 and a mouse 420, and the user can perform operations related to the execution of the measurable suitable feature amount selection method according to the present invention, the display of results, and the like through the operation unit 400.

### <Process of Measurable Suitable Feature Amount Selection Method>

The measurable suitable feature amount selection method according to the present invention is a method for selecting a feature amount that is used for predicting or discriminating features of a sample and that is measurable and includes a data set input step, a feature amount candidate extraction step, a feature amount selection candidate extraction step, a measurement element design step, and a measurement element design result notification step.

The data set input step is a step of inputting a data set of a sample group in which values of a plurality of feature amounts have been recorded. The feature amount candidate extraction step is a step of extracting feature amount candidates from the data set. The feature amount selection candidate extraction step is a step of selecting a feature amount which predicts or discriminates the features of the sample and extracting feature amount selection candidates. The measurement element design step is a step of designing a measurement element that can measure a feature amount and extracting a feature amount, for which the design of the measurement element has succeeded, as the measurable feature amount. The measurement element design result notification step is a step of feeding back the result of whether the design of the measurement element has succeeded or failed in the measurement element design step to the feature amount selection candidate extraction step or notifying the feature amount selection candidate extraction step of the measurable feature amount. The order of the feature amount selection candidate extraction step and the measurement element design step differs depending on each embodiment, and the feature amount selection candidate extraction step is performed on the feature amount candidate extracted in the feature amount candidate extraction step or on the measurable feature amount extracted as the feature amount for which the design of the measurement element has succeeded in the measurement element design step. Further, the measurement element design step is performed on the feature amount candidate extracted in the feature amount candidate extraction step or on the feature amount selection candidate extracted in the feature amount selection candidate extraction step. In the measurement element design result notification step, the success or failure of the design of the measurement element is fed back to the feature amount selection candidate extraction step, or a notification of the measurable feature amount is sent to the feature amount selection candidate extraction step to select the measurable suitable feature amount, for which the measurement element can be designed and which predicts or discriminates the features of the sample, from the feature amount candidates.

Hereinafter, each embodiment of the measurable suitable feature amount selection method will be described. In addition, as an example, a case (particularly, a cancer type) where the feature amount is a gene (particularly, a DNA methylation site), the measurement element is a PCR primer, and multi-class classification is performed on given samples (particularly, tumor tissues) by machine learning will be described below. Further, the measurable suitable feature amount selection method according to the present invention is not limited to the above-described case. The feature amount may be other than the gene, and the measurement element is not limited to the PCR primer and can be determined depending on the feature amount. Other embodiments will be described below. However, the measurable suitable feature amount selection method according to the present invention has a high effect in a case where a success rate of the design of the measurement element is low or in a case where the method is effective in a combination of the feature amounts. Therefore, an example in which the DNA methylation site is used as the feature amount is one of suitable application examples. In addition, the present invention is particularly effective in a case where a plurality of feature amounts are selected. Therefore, the present invention is particularly suitable for multi-class classification problems or relatively complex regression problems, but the application of the present invention is not limited to this case.

### [First Embodiment]

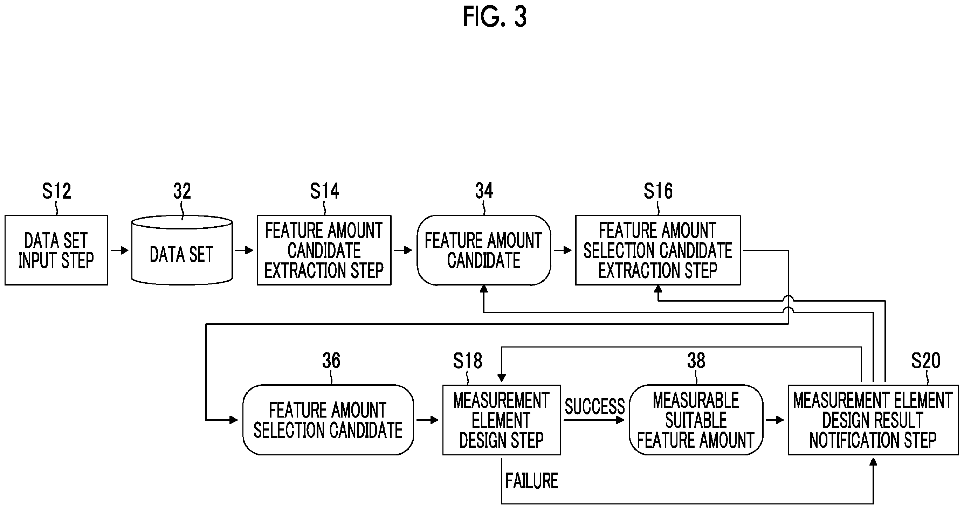

Fig. 3 is a flowchart illustrating a measurable suitable feature amount selection method according to a first embodiment. The measurable suitable feature amount selection method according to the first embodiment includes a data set input step (Step S12), a feature amount candidate extraction step (Step S14), a feature amount selection candidate extraction step (Step S16), a measurement element design step (Step S18), and a measurement element design result notification step (Step S20). In the measurable suitable feature amount selection method according to the first embodiment, in the feature amount selection candidate extraction step (Step S16), one or a plurality of feature amounts are selected from the feature amount candidates, and a feature amount selection candidate is extracted. In the measurement element design step (Step S18), the design of the measurement element is performed on the feature amount selection candidate extracted in the feature amount selection candidate extraction step (Step S16), and the feature amount selection candidate is extracted as a measurable suitable feature amount. In the measurement element design result notification step (Step S20), in a case where it is determined that the design of the measurement element has failed on the basis of the result of the measurement element design step (Step S18), at least one of the following is performed: the feature amount determined as the failure is fed back to the feature amount candidates and is deleted the feature amount candidates; the measurable suitable feature amount is fed back to the feature amount selection candidate extraction step (Step S16), and the conditions of the feature amount selection candidate extraction step (Step S16) are controlled; or the measurable suitable feature amount is fed back to the measurement element design step (Step S18), and the conditions of the measurement element design step (Step S18) are controlled. Then, in the measurement element design step (Step S18), the feature amount selection candidate extraction step (Step S16), the measurement element design step (Step S18), and the measurement element design result notification step (Step S20) are repeated until a desired number of measurable suitable feature amounts can be selected.

Hereinafter, each of the steps will be described.

### <<Data Set Input Step (Step S12)>>

The data set input processing unit 102 of the measurable suitable feature amount selection device 10 performs the data set input step (Step S12). The data set input step (Step S12) is a step of inputting a data set 32 of a sample group in which the values of a plurality of feature amounts have been recorded. The data set 32 is composed of a known sample group that belongs to a given class as a target and a feature amount group of the known sample group. Further, it is assumed that a learning data set is given and every sample is given the values of a plurality of common feature amounts (for example, DNA methylation sites) and one correct answer class label (for example, cancer or non-cancer, and tissue classification) (the learning data set is input by the data set input processing unit 102).

Furthermore, input sample data may be divided into learning data and test data. A cancer type is given as a correct answer label to each sample of the learning data. In addition, each sample has a plurality of DNA methylation sites, and a degree of methylation is given to each of the DNA methylation sites. This degree of methylation can be, for example, a value measured by comprehensive measurement means such as a microarray.

### <<Feature Amount Candidate Extraction Step (Step S14)>>

The feature amount candidate extraction processing unit 104 of the measurable suitable feature amount selection device 10 performs the feature amount candidate extraction step (Step S14). The feature amount candidate extraction step (Step S14) is a step of extracting a feature amount candidate 34 from the data set 32 input in the data set input step (Step S12). Since the data set 32 includes information, such as a sample name and a class label, other than the feature amount, the feature amount is extracted as the feature amount candidate 34 from the data set 32. As a method for extracting the feature amount candidate 34, a method for transcribing or reading out the feature amount from the data set 32 can be used.

### <<Feature Amount Selection Candidate Extraction Step (Step S16)>>

The feature amount selection candidate extraction processing unit 106 of the measurable suitable feature amount selection device 10 performs the feature amount selection candidate extraction step (Step S16). In the feature amount selection candidate extraction step (Step S16), one or a plurality of feature amounts that predict or discriminate the features of the sample are selected as feature amount selection candidates 36 from the feature amount candidates 34.

Feature amounts that can be classified into multiple cancer classes is selected as the feature amounts (feature amount selection candidate 36). In the feature amount selection candidate extraction step (Step S16), the feature amounts that can be efficiently classified into multiple cancer classes are selected with reference to a cancer type and the degree of DNA methylation in each sample of the learning data. The feature amount selection candidate can be selected by a known method. A so-called filter method, wrapper method, or embedded method may be used. Specifically, for example, any of various methods introduced in papers, such as "A review of feature selection techniques in bioinformatics" (Yvan Saeys, 2007: Bioinformatics), may be used. In particular, the effect of the present invention is high in a case where a method in which a combination of feature amounts is effective is applied. For example, the Sequential Backward Elimination approach of the wrapper method is a method that first selects all feature amounts and then removes the feature amounts with the least degree of deterioration in class classification performance one by one and in which a combination of the feature amounts is effective. As another example, the method described in WO2021/161901A, which is an application by the applicant, may be applied.

### <<Measurement Element Design Step (Step S18)>>

The measurement element design processing unit 108 of the measurable suitable feature amount selection device 10 performs the measurement element design step (Step S18). In the measurement element design step (Step S18), the measurement element is designed for the feature amount selection candidate 36, and the feature amount for which the design of the measurement element has succeeded is extracted as a measurable suitable feature amount 38.

The measurement element is designed for the feature amount selection candidate 36 extracted in the feature amount selection candidate extraction step (Step S16). The PCR primer that can measure the DNA methylation site, which is gene information of the extracted feature amount selection candidate 36, is designed. The PCR primer (hereinafter, referred to as a "primer") is a synthetic oligo DNA having a length of several tens of bases. A pair of PCR primers complementarily bind to the vicinity of the methylation site such that a target methylation site (hereinafter, referred to as a "target") is interposed therebetween. However, the primer needs to specifically bind to the end of the target. That is, it is necessary to suppress the possibility that the primer will bind to DNA other than the target. In addition, for the primer, it is necessary to control the reaction temperature called a Tm value (temperature at which 50% of double-stranded DNA dissociates into single-stranded DNA. Tm is derived from the melting temperature) which is determined by the number of bases, the arrangement of bases, and the like, within a predetermined range. Further, it is necessary to control a GC ratio (means the total molar percentage of guanine (G) and cytosine (C) in all nucleic acid bases) within a predetermined range. Moreover, it is also necessary to suppress the complementarity between the primers such that a by-product called a primer dimer is not generated due to the binding of any primers. In addition, in some cases, many known conditions are required for the primer. For the design of the primer, it is necessary to not only design the primer that binds to the target as described above but also consider the relationship with other primers in the design.

Further, for the design of the primer, in some cases, it is not possible to reliably design the primer that binds to the end of the target, and the success rate of the design of the primer is low. For example, in the measurement of the DNA methylation sites, a pretreatment called bisulfite conversion is performed. In the bisulfite conversion, a chemical treatment is applied in which unmethylated cytosine (unmethylated cytosine) in DNA is converted into uracil, but methylated cytosine is not converted. This makes it possible to distinguish the methylated cytosine from the unmethylated cytosine. However, since it is not possible to determine in advance whether or not each site of the target is methylated, for example, it is necessary to consider that cytosine is not included in the primer. Therefore, it may be difficult to design an appropriate primer depending on the target.

As the primer design method, a known method can be used. For example, the primer design method described in JP6475321B which is an application by the applicant can be applied.

Further, in the present invention, the "design" may include a process up to the determination of the base sequence of the primer. In addition, in some cases, there is a problem that cannot be solved by the prior examination. Therefore, the design may include a process up to confirming that the feature amounts of the sample can be measured by experiments.

In the measurable suitable feature amount selection method according to the first embodiment, in the next measurement element design result notification step (Step S20), the result of whether the design of the measurement element has succeeded or failed is fed back to each data item or each step such that the measurable suitable feature amount 38 for which the measurement element can be designed and which predicts or discriminates the features of the sample is selected.

### <<Measurement Element Design Result Notification Step (Step S20)>>

The measurement element design result notification processing unit 110 of the measurable suitable feature amount selection device 10 feeds back the result of whether the design of the measurement element has succeeded or failed in the measurement element design step (Step S18).

In the measurement element design result notification step (Step S20), at least one of the following is performed: (i) in a case where it is determined that the design of the measurement element has failed on the basis of the result of the measurement element design step (Step S18), the feature amount determined as the failure is fed back to the feature amount candidates 34 and is deleted from the feature amount candidates 34; (ii) the measurable suitable feature amount 38 is fed back to the feature amount selection candidate extraction step (Step S16), and the conditions of the feature amount selection candidate extraction step (Step S16) are controlled; or (iii) the measurable suitable feature amount 38 is fed back to the measurement element design step (Step S20), and the conditions of the measurement element design step (Step S20) are controlled.

The feature amount for which the design of the measurement element is determined to fail is deleted from the feature amount candidates 34. Therefore, in the feature selection candidate extraction step (Step S16), the selection of the feature amount for which the measurement element is not capable of being designed is prevented. Therefore, it is possible to increase the possibility that the feature amount extracted in the feature selection candidate extraction step (Step S16) will be the feature amount for which the measurement element can be designed.

In addition, the control of the conditions of the feature selection candidate extraction step (Step S16) on the basis of the extracted measurable suitable feature amount 38 makes it possible to select the feature amount selection candidate 36 in consideration of the measurable suitable feature amount 38 that has already been confirmed. For example, the feature amount selection candidate 36 can be extracted in consideration of a combination of the feature amounts, by evaluating the combination of the feature amounts, calculating a certain evaluation value, and extracting the feature amount selection candidate 36 on the basis of the evaluation value. In this case, in the feature amount selection candidate extraction step (Step S16), in a case where a certain feature amount is added to the measurable suitable feature amount 38 by greedy search, one or a small number of feature amounts having a high selection priority whose evaluation value is most improved are specified, and this feature amount is used as the feature amount selection candidate 36. Therefore, it can be said that the selected feature amount selection candidate 36 is a feature amount that is combined with the already selected measurable suitable feature amount 38 to exhibit an effect. The evaluation value used to evaluate the combination can be determined by the method of the feature amount selection candidate extraction step (Step S16). For example, the feature amount selection candidate that is effective in the class classification performance can be extracted by the wrapper method.

Further, the control of the conditions of the measurement element design step (Step S18) on the basis of the extracted measurable suitable feature amount makes it possible to design a measurement element for the next feature amount selection candidate 36 in consideration of the measurable suitable feature amount 38 that has already been confirmed. It is possible to select the additional measurable suitable feature amount 38 in consideration of interaction with a set of the measurement elements designed for the measurable suitable feature amounts 38. An example of the interaction with the set of the measurement elements is the formation of dimers between the primers.

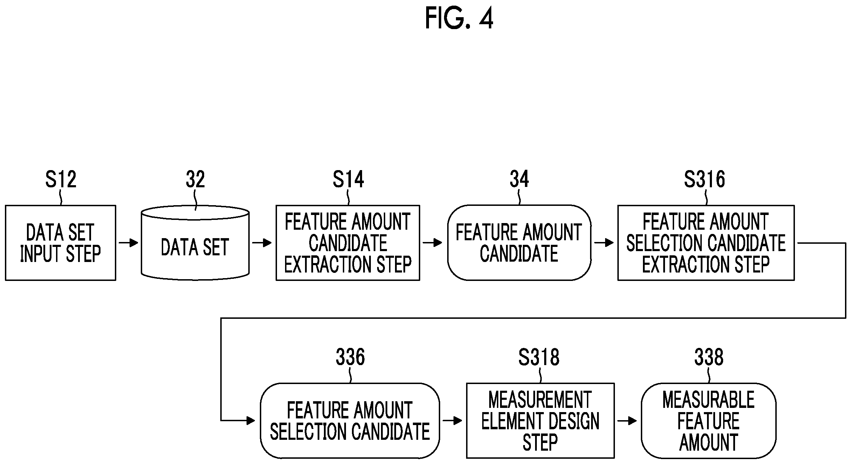

Fig. 4 illustrates a measurable feature amount selection method according to the related art which does not consider the success rate of the design of the primer. In the method according to the related art, similarly, the feature amount candidate 34 is extracted by the data set input step (Step S12) and the feature amount candidate extraction step (Step S14). In the selection method according to the related art, a feature amount selection candidate extraction step (Step S316) is performed on all or a large number of the feature amounts of the feature amount candidates 34 to extract feature amount selection candidates 336. Then, a measurement element design step (Step S318) is performed on the feature amount selection candidates 336 to extract a measurable feature amount 338. In this case, the measurable feature amount 338 can be extracted. However, the measurable feature amount 338 may not be extracted in consideration of the effect of the combination of the feature amounts evaluated in the feature amount selection candidate extraction step (Step S316). In addition, the feature amount selection candidates 336 extracted in the feature amount selection candidate extraction step (Step S316) also include a feature amount having a low success rate of the design of the measurement element. In some cases, it is not possible to design the measurement element even in a case where the feature amounts are selected in consideration of the combination.

In the measurable suitable feature amount selection method according to the first embodiment, the feature amount selection candidate 36 for which the measurement element can be designed and the feature amount selection candidate 36 considering the combination of the feature amounts in the feature amount selection candidate extraction step (Step S16) and the measurable suitable feature amount 38 considering the interaction with the set of the measurement elements in the measurement element design step (Step S18) can be selected. Therefore, it is possible to select the measurable suitable feature amount 38 for which the measurement element can be designed and which predicts or discriminates the features of the sample. Further, since one or a small number of feature amounts are selected from the feature amount candidates 34 in the feature amount selection candidate extraction step (Step S16), the result of the measurement element design step (Step S18) for the feature amount is fed back to enable flexible applications to various cases.

### [Second Embodiment]

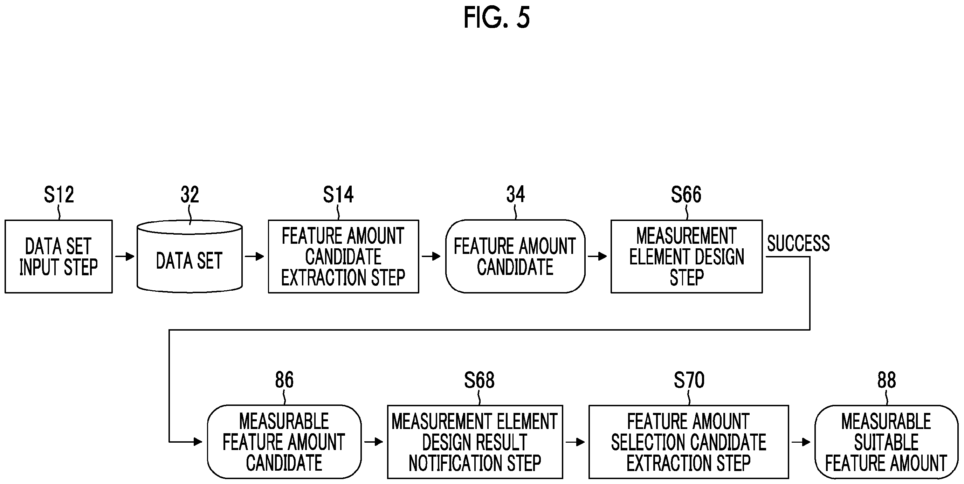

Fig. 5 is a flowchart illustrating a measurable suitable feature amount selection method according to a second embodiment. The measurable suitable feature amount selection method according to the second embodiment includes the data set input step (Step S12), the feature amount candidate extraction step (Step S14), a measurement element design step (Step S66), a measurement element design result notification step (Step S68), and a feature amount selection candidate extraction step (Step S70). In the measurable suitable feature amount selection method according to the second embodiment, after the feature amount candidate extraction step (Step S14) is performed, the measurement element design step (Step S66) is performed on the feature amount candidates 34, and the feature amount for which the design of the measurement element is determined to succeed is extracted as a measurable feature amount candidate 86. In the measurement element design result notification step (Step S68), a notification of the measurable feature amount candidate 86 is sent to the feature amount selection candidate extraction step (Step S70). In the feature amount selection candidate extraction step (Step S70), the extraction is performed until a desired number of measurable suitable feature amounts 88 can be selected from the measurable feature amount candidates 86.

### «Data Set Input Step (Step S12) and Feature Amount Candidate Extraction Step (Step S 14)»

Since the data set input step (Step S12) and the feature amount candidate extraction step (Step S14) can be performed in the same manner as those in the measurable suitable feature amount selection method according to the first embodiment, the description thereof will not be repeated.

### <<Measurement Element Design Step (Step S66)>>

In the measurement element design step (Step S66), the measurement element is designed for the feature amount candidate 34, and the feature amount for which the design of the measurement element has succeeded is extracted as the measurable feature amount candidate 86. The measurement element design step (Step S66) can be performed by the same method as the measurement element design step (Step S18) according to the first embodiment.

The measurement element design step (Step S66) is performed on all of the feature amount candidates 34. In addition, strictly speaking, the measurement element design step may not be performed on all of the feature amount candidates 34. For example, in a case where there are about 10,000 feature amount candidates 34, the measurement element design process may be performed except for a few feature amount candidates at the end. The same applies to the following embodiments.

### <<Measurement Element Design Result Notification Step (Step S68)>>

In the measurement element design result notification step (Step S68), a notification of the measurable feature amount candidate 86 is set to the feature amount selection candidate extraction step (Step S70). The measurable feature amount candidate 86 is extracted by the measurement design step (Step S66), and the extracted feature amount is a feature amount for which the measurement element can be designed and which is extracted in consideration of the interaction with a set of the designed measurement elements.

### <<Feature Amount Selection Candidate Extraction Step (Step S70)>>

In the feature amount selection candidate extraction step (Step S70), for the measurable feature amount candidates 86, a feature amount that predicts or discriminates the features of the sample is extracted as the measurable suitable feature amount 88. The feature amount selection candidate extraction step (Step S70) can be performed by the same method as the feature amount selection candidate extraction step (Step S16) according to the first embodiment.

The measurable feature amount candidate 86 is a feature amount for which the measurement element can be designed and which is extracted in consideration of the interaction with the set of the designed measurement elements. The feature amount selection candidate extraction step (Step S70) can be performed on the measurable feature amount candidates 86 to extract the feature amounts that are effective in a combination of the feature amounts. In addition, the selected feature amount is a feature amount for which the measurement element can be designed. Therefore, for the feature amounts extracted in the feature amount selection candidate extraction step (Step S70), the measurable suitable feature amount 88 for which the measurement element can be designed and which predicts or discriminates the features of the sample is selected.

In the feature amount selection candidate extraction step (Step S70), the extraction is performed until a desired number of measurable suitable feature amounts 88 can be selected.

In the measurable suitable feature amount selection method according to the second embodiment, the feature amount selection candidate extraction step (Step S70) is performed on all of the measurable feature amount candidates 86 for which the design of the measurement element has already succeeded. Therefore, it is possible to select the measurable suitable feature amount 88 for which the measurement element can be designed and which predicts or discriminates the features of the sample. Further, in the feature amount selection candidate extraction step (Step S70), it is possible to select the best feature amount within the range of the feature amounts for which the design of the measurement element has succeeded. In the measurement element design step (Step S68), the measurement element is designed for all of the feature amount candidates 34. Therefore, the measurement element design step (Step S68) is particularly effective in a case where a measurement element design cost is low. Further, in the present invention, examples of the cost include a required time, a memory consumption, an actual cost. However, the cost is not limited thereto. The same also applies to the following embodiments.

### [Third Embodiment]

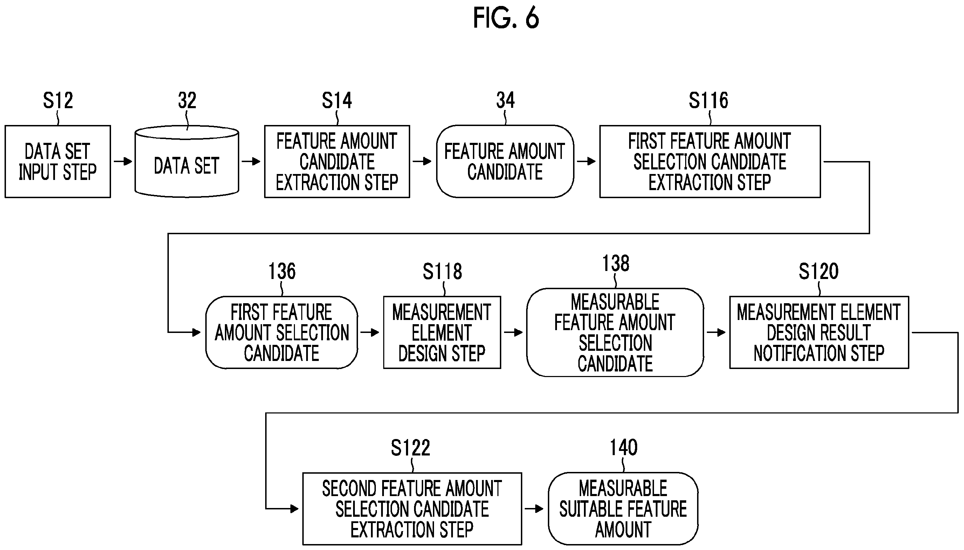

Fig. 6 is a flowchart illustrating a measurable suitable feature amount selection method according to a third embodiment. The measurable suitable feature amount selection method according to the third embodiment includes the data set input step (Step S12), the feature amount candidate extraction step (Step S14), a first feature amount selection candidate extraction step (Step S 1 16), a measurement element design step (Step S 118), a measurement element design result notification step (Step S120), and a second feature amount selection candidate extraction step (Step S122). The measurable suitable feature amount selection method according to the third embodiment includes the first feature amount selection candidate extraction step (Step S 116) of extracting feature amount selection candidates with first feature amount selection means having a weak constraint and the second feature amount selection candidate extraction step (Step S122) of extracting feature amount selection candidates with second feature amount selection means having a strong constraint. The first feature amount selection candidate extraction step (Step S 116) is performed on the feature amount candidates 34 to extract first feature amount selection candidates 136. The measurement element design step (Step S118) is performed on the first feature amount selection candidates 136 to extract a feature amount, for which the design of the measurement element is determined to succeed, as a measurable feature amount selection candidate 138. In the measurement element design result notification step (Step S120), a notification of the measurable feature amount selection candidate 138 is sent to the second feature amount selection candidate extraction step (Step S122). In the second feature amount selection candidate extraction step (Step S122), the extraction is performed until a desired number of measurable suitable feature amounts 140 can be selected from the measurable feature amount selection candidates 138.

### <<Data Set Input Step (Step S12) and Feature Amount Candidate Extraction Step (Step S14)>>

Since the data set input step (Step S12) and the feature amount candidate extraction step (Step S14) can be performed in the same manner as those in the measurable suitable feature amount selection method according to the first embodiment, the description thereof will not be repeated.

### <<First Feature Amount Selection Candidate Extraction Step (Step S116)>>

In the first feature amount selection candidate extraction step (Step S 116), for all of the feature amount candidates 34, the feature amount which predicts or discriminates the features of the sample is extracted as the first feature amount selection candidate 136 by the first feature amount selection means having a weak constraint.

A low-cost method, for example, a simple filter method that depends on the statistics of only feature amount data can be used as the first feature amount selection means having a weak constraint. A clearly unsuitable feature amount is excluded by the first feature amount selection candidate extraction step (Step S116). This makes it possible to narrow down and decrease the number of feature amounts to be subjected to the subsequent measurement element design step (Step S118) from the feature amount candidates 34 to some extent and thus to reduce the number of feature amounts to be subjected to the measurement element design step (Step S118).

### <<Measurement Element Design Step (Step S118)>>

In the measurement element design step (Step S118), the measurement element is designed for the first feature amount selection candidate 136, and a feature amount for which the design of the measurement element has succeeded is extracted as the measurable feature amount selection candidate 138. The measurement element design step (Step S118) can be performed by the same method as the measurement element design step (Step S18) according to the first embodiment. As measurement element design means used in the measurement element design step (Step S118), it is preferable to use measurement element design means with an intermediate cost for the first feature amount selection means having a weak constraint and the second feature amount selection means having a strong constraint. However, the cost of the measurement element design means may be low or high and is not particularly limited.

### <<Measurement Element Design Result Notification Step (Step S120)>>

In the measurement element design result notification step (Step S120), a notification of the measurable feature amount selection candidate 138 is sent to the second feature amount selection candidate extraction step (Step S122). The measurable feature amount selection candidate 138 is extracted in the measurement design step (Step S118), and the extracted feature amount is a feature amount for which the measurement element can be designed and which is extracted in consideration of the interaction with a set of the designed measurement elements.

### <<Second Feature Amount Selection Candidate Extraction Step (Step S122)>>

In the second feature amount selection candidate extraction step (Step S122), for the measurable feature amount selection candidates 138, the feature amount which predicts or discriminates the features of the sample is extracted and confirmed as a measurable suitable feature amount 140 by the second feature amount selection means having a high constraint.

A high-cost method, for example, a wrapper method can be used as the second feature amount selection means having a high constraint.

The measurable feature amount selection candidate 138 is a feature amount for which the measurement element can be designed and which is extracted in consideration of the interaction with the set of the designed measurement elements. The second feature amount selection candidate extraction step (Step S122) can be performed on the measurable feature amount selection candidates 138 to extract the feature amounts that are effective in a combination of the feature amounts. In addition, the selected feature amount is a feature amount for which the measurement element can be designed. Therefore, for the feature amounts extracted by the second feature amount selection candidate extraction step (Step S122), the measurable suitable feature amount 140 for which the measurement element can be designed and which predicts or discriminates the features of the sample is selected.

In the second feature amount selection candidate extraction step (Step S122), the extraction is performed until a desired number of measurable suitable feature amounts 140 can be selected.

In the measurable suitable feature amount selection method according to the third embodiment, the second feature amount selection candidate extraction step (Step S122) is performed on all of the measurable feature amount selection candidates 138 for which the design of the measurement element has already succeeded. Therefore, it is possible to select the measurable suitable feature amount 140 for which the measurement element can be designed and which predicts or discriminates the features of the sample. Since the first feature amount selection candidate extraction step (Step S116) and the second feature amount selection candidate extraction step (Step S122) are sequentially applied from the lowest cost method, it is possible to suppress a cost and to select the measurable suitable feature amount 140 which is a feature amount for which the measurement element can be designed more easily.

Further, in the flowchart illustrated in Fig. 6, the second feature amount selection candidate extraction step (Step S122) is performed on the measurable feature amount selection candidates 138 extracted by the first feature amount selection candidate extraction step (Step S116) and the measurement element design step (Step S118). However, the second feature amount selection candidate extraction step (step S122) may be performed on the measurable suitable feature amount obtained by the measurable suitable feature amount selection method according to the first embodiment to further narrow down the feature amounts.

### [Fourth Embodiment]

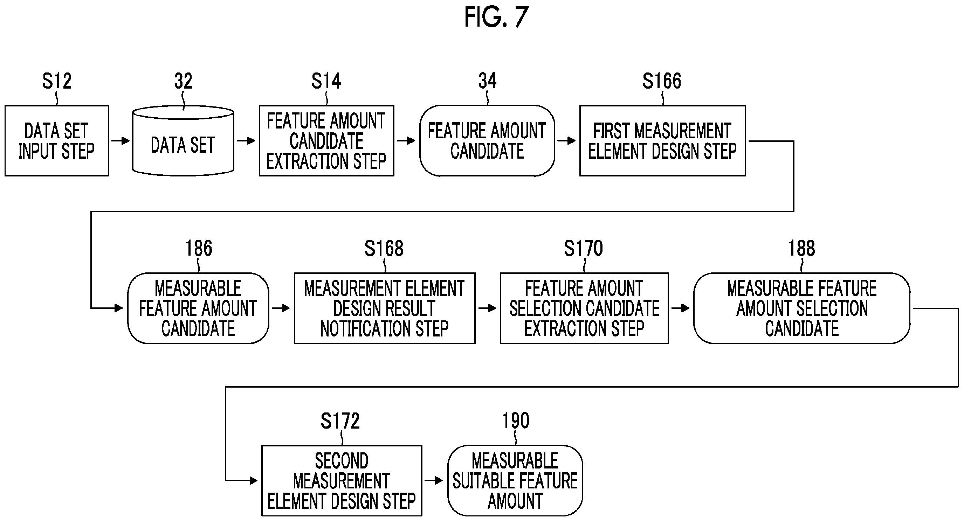

Fig. 7 is a flowchart illustrating a measurable suitable feature amount selection method according to a fourth embodiment. The measurable suitable feature amount selection method according to the fourth embodiment includes the data set input step (Step S12), the feature amount candidate extraction step (Step S14), a first measurement element design step (Step S166), a measurement element design result notification step (Step S168), a feature amount selection candidate extraction step (Step S170), and a second measurement element design step (Step S172). The measurable suitable feature amount selection method according to the fourth embodiment includes the first measurement element design step (Step S166) of designing the measurement element with first measurement element design means having a weak constraint and the second measurement element design step (Step S172) of designing the measurement element with second measurement element design means having a strong constraint. The first measurement element design step (Step S166) is performed on the feature amount candidates 34 to extract the feature amount, for which the design of the measurement element is determined to succeed, as a measurable feature amount candidate 186. In the measurement element design result notification step (Step S168), a notification of the measurable feature amount candidate 186 is sent to the feature amount selection candidate extraction step (Step S170). The feature amount selection candidate extraction step (Step S170) is performed on the measurable feature amount candidates 186 to extract measurable feature amount selection candidates 188. The second measurement element design step (Step S172) is performed on the measurable feature amount selection candidates 188 to extract the feature amount, for which the design of the measurement element is determined to succeed, as a measurable suitable feature amount 190. The extraction is performed until a desired number of measurable suitable feature amounts 190 can be selected.

### <<Data Set Input Step (Step S 12) and Feature Amount Candidate Extraction Step (Step S14)>>

Since the data set input step (Step S12) and the feature amount candidate extraction step (Step S14) can be performed in the same manner as those in the measurable suitable feature amount selection method according to the first embodiment, the description thereof will not be repeated.

### <<First Measurement Element Design Step (Step S166)>>

In the first measurement element design step (Step S166), the measurement element is designed for all of the feature amount candidates 34 by the first measurement element design means having a weak constraint, and the feature amount for which the design of the measurement element is determined to succeed is extracted as the measurable feature amount candidate 186.

A low-cost method, for example, a method for determining whether or not the design can be performed with only the primer can be performed as the first measurement element design means having a weak constraint. In this case, the number of feature amounts is narrowed down and reduced. This makes it possible to reduce the number of feature amounts to be subjected to the feature amount selection candidate extraction step (Step S 170).

### <<Measurement Element Design Result Notification Step (Step S168)>>

In the measurement element design result notification step (Step S168), a notification of the measurable feature amount candidate 186 is sent to the feature amount selection candidate extraction step (Step S170). The measurable feature amount candidate 186 is extracted by the first measurement design step (Step S166), and the extracted feature amount is a feature amount for which the measurement element can be designed.

### <<Feature Amount Selection Candidate Extraction Step (Step S170)>>

In the feature amount selection candidate extraction step (Step S170), for the measurable feature amount candidates 186, a feature amount that predicts or discriminates the features of the sample is extracted as the measurable feature amount selection candidate 188. The feature amount selection candidate extraction step (Step S170) can be performed by the same method as the feature amount selection candidate extraction step (Step S16) according to the first embodiment. As the feature amount selection means used in the feature amount selection candidate extraction step (Step S170), it is preferable to use feature amount selection means with an intermediate cost for the first measurement element design means having a weak constraint and the second measurement element design means having a strong constraint. The cost of the feature amount selection means may be low or high and is not particularly limited.

### <<Second Measurement Element Design Step (Step S172)>>

In the second measurement element design step (Step S172), the measurement element is designed for the measurable feature amount selection candidate 188 by the second measurement element design means having a high constraint, and a feature amount for which the design of the measurement element is determined to succeed is extracted and confirmed as the measurable suitable feature amount 190.

A high-cost method, for example, means including the evaluation of the interaction of the primer, such as the evaluation of a primer dimer, can be used as the second measurement element design means having a high constraint.

The measurable feature amount selection candidate 188 is a feature amount for which the measurement element can be designed and which is effective in a combination of the feature amounts. The second measurement element design step (Step S172) can be performed on the measurable feature amount selection candidates 188 to extract a feature amount in consideration of the interaction of a set of the measurement elements. Therefore, for the feature amounts extracted by the second measurement element design step (Step S172), the measurable suitable feature amount 190, for which the measurement element can be designed and which predicts or discriminates the features of the sample, is selected.

In the second measurement element design step (Step S172), the extraction is performed until a desired number of measurable suitable feature amounts 190 can be selected.

In the measurable suitable feature amount selection method according to the fourth embodiment, the second measurement element design step (Step S172) is performed on all of the measurable feature amount selection candidates 188 for which the design of the measurement element has already succeeded. Therefore, it is possible to select the measurable suitable feature amount 190 for which the measurement element can be designed and which predicts or discriminates the features of the sample. Since the first measurement element design step (Step S166) and the second feature amount selection candidate extraction step (Step S172) are sequentially applied from the lowest cost method, it is possible to suppress a cost and to select the measurable suitable feature amount 190 which is a feature amount for which the measurement element can be designed more easily.

Further, in the flowchart illustrated in Fig. 7, after the first measurement element design step (Step S166) is performed, the feature amount selection candidate extraction step (Step S170) and the second measurement element design step (Step S172) are performed. However, after the first measurement element design step (Step S166) is performed, the measurable suitable feature amount selection method according to the first embodiment may be performed to extract the measurable suitable feature amount 190. In this case, the measurement element design step (Step S18) (corresponding to the second measurement element design step (Step S172)) performed in the measurable suitable feature amount selection method according to the first embodiment is performed by the second measurement element design means having a stronger constraint than the first measurement element design means performed in the first measurement element design step (Step S166). Further, the second feature amount selection candidate extraction step may be performed on the obtained measurable suitable feature amount 190 to further narrow down the feature amounts.

### [Fifth Embodiment]

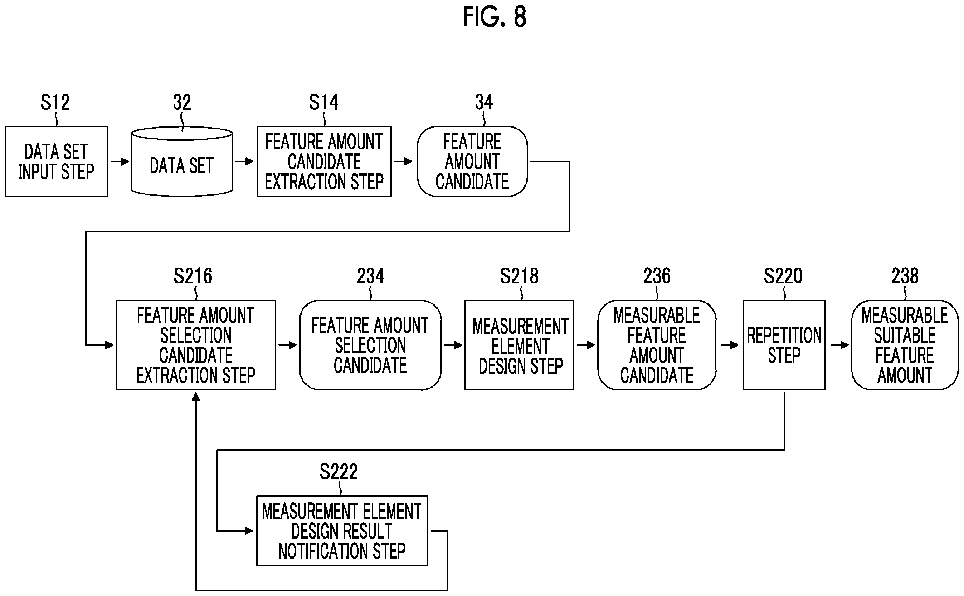

Fig. 8 is a flowchart illustrating an example of a measurable suitable feature amount selection method according to a fifth embodiment. The measurable suitable feature amount selection method according to the fifth embodiment includes the data set input step (Step S12), the feature amount candidate extraction step (Step S14), a feature amount selection candidate extraction step (Step S216), a measurement element design step (Step S218), a repetition step (Step S220), and a measurement element design result notification step (Step S222). The repetition step (Step S220) is a step of optionally repeating the feature amount selection candidate extraction step (Step S216) and the measurement element design step (Step S218). The repetition step (Step S220) is performed to select a measurable suitable feature amount 238, for which the measurement element can be designed, in stages.

### <<Data Set Input Step (Step S12) and Feature Amount Candidate Extraction Step (Step S14)>>

Since the data set input step (Step S12) and the feature amount candidate extraction step (Step S14) can be performed in the same manner as those in the measurable suitable feature amount selection method according to the first embodiment, the description thereof will not be repeated.

### <<Feature Amount Selection Candidate Extraction Step (Step S216)>>

In the feature amount selection candidate extraction step (Step S216), for all of the feature amount candidates 34, the feature amount which predicts or discriminates the features of the sample is extracted as a feature amount selection candidate 234. The feature amount selection candidate extraction step (Step S216) can be performed by the same method as the feature amount selection candidate extraction step (Step S16) according to the first embodiment.

### <<Measurement Element Design Step (Step S218)>>

In the measurement element design step (Step S218), the measurement element is designed for the feature amount selection candidate 234, and the feature amount for which the design of the measurement element has succeeded is extracted as a measurable feature amount candidate 236. The measurement element design step (Step S218) can be performed by the same method as the measurement element design step (Step S18) according to the first embodiment.

### <<Repetition Step (Step S220) and Measurement Element Design Result Notification Step (Step S222)>>

In the repetition step (Step S220), the feature amount selection candidate extraction step (Step S216) and the measurement element design step (Step S218) are optionally repeated. Further, in a case where it is determined that the feature amount selection candidate extraction step (Step S216) and the measurement element design step (Step S218) are repeated in the repetition step (Step S220), in the measurement element design result notification step (Step S222), a notification of the measurable feature amount candidate 236 is sent to the feature amount selection candidate extraction step (Step S216). In the feature amount selection candidate extraction step (Step S216), the feature amount selection candidate 234 is extracted for the notified measurable feature amount candidates 236. Then, the repetition step (Step S220) of repeating the feature amount selection candidate extraction step (Step S216) and the measurement element design step (Step S218) is performed until a desired number of measurable feature amount candidates 236 can be extracted.

For the measurable feature amount candidates 236 extracted by repeating the feature amount selection candidate extraction step (Step S216) and the measurement element design step (Step S218) in the repetition step (Step S220), a feature amount, for which the measurement element can be designed and which is selected in consideration of a combination of the feature amounts and the interaction with a set of the measurement elements, can be extracted as the measurable suitable feature amount 238. In addition, in a case where the repetition step (Step S220) is performed, the measurement element design step (Step S218) is performed to extract the measurable feature amount candidate 236 before the repetition step (Step S220) is performed as the measurable suitable feature amount 238. Therefore, it is possible to extract the measurable suitable feature amount 238 in stages.

It is preferable that the feature amount selection candidate extraction step (Step S216) repeated by the repetition step (Step S220) is performed in ascending order of constraint. In addition, it is preferable that the measurement element design step (Step S218) is also performed in ascending order of constraint.

In the measurable suitable feature amount selection method according to the fifth embodiment, while a notification of the measurable feature amount candidate 236 for which the design of the measurement element has succeeded is sent to the feature amount selection candidate extraction step (Step S216) in the measurement element design result notification step (Step S222), the feature amount selection candidate extraction step (Step S216) and the measurement element design step (Step S218) can be repeated to extract the feature amount, for which the measurement element can be design and which is selected in consideration of a combination of the feature amounts and the interaction with a set of the measurement elements, as the measurable suitable feature amount 238.

In the measurable suitable feature amount selection method according to the fifth embodiment, in the flowchart illustrated in Fig. 8, the feature amount selection candidate extraction step (Step S216) is performed on the feature amount candidate 34. However, the measurement element design step (Step S218) may be performed first. Further, instead of alternately performing the feature amount selection candidate extraction step (Step S216) and the measurement element design step (Step S218), one of the two steps may be continuously performed.

### [Other Application Examples]

The aspect in which the feature amount is a gene, the gene is the information of the DNA methylation site, and the measurement element is the primer which is gene measurement means has been described above. However, the present invention is not limited thereto. A biomarker, such as DNA mutation, mRNA, miRNA, a protein, or a metabolite can be used as the gene. In addition, a probe can be used as the measurement element.

Furthermore, the present invention can also be applied to the following problems.
(1) Design of diagnosis: in a problem for the purpose of detecting a disease, medical examination items are used as the feature amounts, and examination means that can be implemented in any diagnosis form, such as a health checkup, among the feature amounts is used as the measurement element. For example, it may be determined whether or not the examination means (measurement element) can be designed according to a required examination skill level, an examination cost, an examination time, and the like.
(2) Consideration of privacy and the like: in a problem for the purpose of estimating personal preference and the like, a questionnaire and a personal attribute are used as the feature amounts, and, for example, acquisition means that can be implemented by privacy and related regulations among the feature amount is used as the measurement element. For example, it may be determined whether or not the acquisition means (measurement element) can be designed according to a required regulation level, an acquisition cost, an acquisition time, the response rate of the questionnaire, and the like.
(3) Drone imaging: in a problem of determining the state of a building and the like, captured images are used as the feature amounts, and an image that can be captured by, for example, a drone among the feature amounts is used as the measurement element. For example, it may be determined whether or not imaging means (measurement element) can be designed according to the number of consecutively captured images, the difficulty of imaging, regulations, and the like.

Since each of the above aspects is embodied in an executable form, it is clear that the present invention can be similarly applied to each problem or a wide general problem only by replacing the specific representations of the feature amount and the measurement element. In addition, whether or not the measurement element can be designed may be determined not only on the basis of technical difficulty but also on the basis of regulations, financial or time costs, a success rate of acquisition, and the like. In any case, the selected feature amount can be flexibly configured by an examination format, privacy regulations, drone performance, related regulations, or the like on the basis of a certain master data set, or seamless development is possible in a case where the measurement element is switched by a study on practical use after a feasibility study.

### Examples

Hereinafter, the present invention will be described in more detail with reference to examples of the present invention.

An example will be described in which the feature amount is a DNA methylation site, the measurement element is a PCR primer targeting each DNA methylation site, and a given tumor tissue sample is subjected to multi-class classification by machine learning.

Eight types of cancers, such as colorectal cancer, stomach cancer, lung cancer, breast cancer, prostate cancer, pancreatic cancer, liver cancer, and cervical cancer, were selected as the objects to be diagnosed. Further, in addition to the organs corresponding to these cancers, a total of 24 possible types including blood, kidney, thyroid, and the like were listed. For these cancers, 5,110 samples of DNA methylation sites were collected. For the cancer tumor and the normal organs (excluding blood), 4,378 samples were collected from the registered data of "The Cancer Genome Atlas" (TCGA) [Tomczak, Katarzyna, et al., 2015]. In addition, for blood, 732 samples were also collected from a separate data set [Johansson, Asa, Stefan Enroth, and Ulf Gyllensten, 2013]. All of the classes to which the samples belonged (origin tissues including distinction between cancer and non-cancer) were given according to the registered annotation information. In addition, approximately 2,000 DNA methylation sites (all feature amounts) were used.

The feature amount selection and multi-class classification method described in WO2021/161901A which was an application by the applicant was applied to an input data set. In addition, the design of the primer was simulated with a setting in which the design would fail with a given probability (10%), and multi-class classification performance (average F-measure) was measured in each of the following cases.
(1) Comparative Example 1: As a method corresponding to the method according to the related art, a selection priority order was determined in advance for all of 2,000 feature amounts by feature amount selection, the success or failure of the design of the measurement element for each feature amount was sequentially determined at random on the basis of a given probability, and a total of 25 feature amounts were selected.
(2) Example 1: A total of 25 measurable suitable feature amounts were selected by the method described in the first embodiment while the success or failure of the design of the measurement element for each feature amount was sequentially determined at random on the basis of a given probability.
(3) Example 2: By the method described in the second embodiment, a measurement element that could be designed in advance was randomly determined on the basis of a given probability, and a total of 25 measurable suitable feature amounts were selected therefrom.

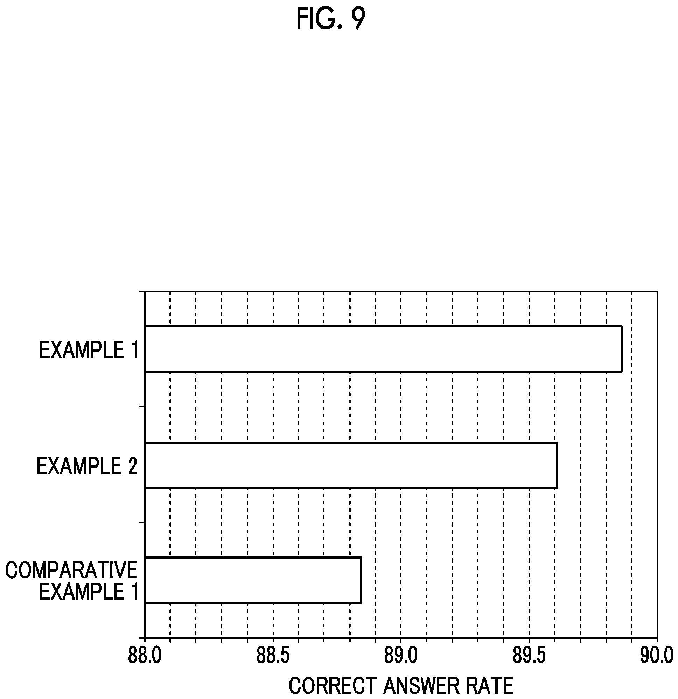

Table 1 and Fig. 9 show comparison among the multi-class classification performance (overall correct answer rate) results of Comparative Example 1 and Examples 1 and 2. In addition, a numerical value is an average value of 30 random trials.

**[Table 1]**

| | Comparative Example 1 | Example 1 | Example 2 |
|---|---|---|---|
| Correct answer rate | 0.888454 | 0.898591 | 0.896086 |

Both Examples 1 and 2 showed better results than the related art (Comparative Example 1).

Further, for the third to fifth embodiments, since the third to fifth embodiments are combinations of the first embodiment and the second embodiment, it is considered that the same results as those in Examples 1 and 2 are obtained. Furthermore, it is clear that a reduction in cost, such as a reduction in processing time, can be achieved according to the costs of feature amount selection and measurement element design by particularly narrowing down the number of feature amount selections to be processed or the number of measurement elements designed in stages.

### Explanation of References

10: measurable suitable feature amount selection device
32: data set
34: feature amount candidate
36: feature amount selection candidate
38: measurable suitable feature amount
86: measurable feature amount candidate
88: measurable suitable feature amount
100: processing unit
102: data set input processing unit
104: feature amount candidate extraction processing unit
106: feature amount selection candidate extraction processing unit
108: measurement element design processing unit
110: measurement element design result notification processing unit
112: output processing unit
114: CPU
116: ROM
118: RAM
136: first feature amount selection candidate
138: measurable feature amount selection candidate
140: measurable suitable feature amount
186: measurable feature amount candidate
188: measurable feature amount selection candidate
190: measurable suitable feature amount
200: storage unit
234: feature amount selection candidate
236: measurable feature amount candidate
238: measurable suitable feature amount
300: display unit
310: monitor
400: operation unit
410: keyboard
420: mouse
500: external server
510: external database
NW: network

## Claims

1. A measurable suitable feature amount selection method for selecting a feature amount that is used to predict or discriminate features of a sample and is measurable, the measurable suitable feature amount selection method comprising:
a data set input step of inputting a data set of a sample group in which values of a plurality of the feature amounts are recorded;
a feature amount candidate extraction step of extracting a feature amount candidate from the data set;
a feature amount selection candidate extraction step of selecting a feature amount for predicting or discriminating the features of the sample and extracting a feature amount selection candidate;
a measurement element design step of designing a measurement element that is capable of measuring the feature amount and extracting a feature amount, for which the design of the measurement element has succeeded, as a measurable feature amount; and
a measurement element design result notification step of feeding back a result of whether the design of the measurement element has succeeded or failed in the measurement element design step to the feature amount selection candidate extraction step or notifying the feature amount selection candidate extraction step of the measurable feature amount,
wherein the feature amount selection candidate extraction step is performed on the feature amount candidate or the measurable feature amount, and the measurement element design step is performed on the feature amount candidate or the feature amount selection candidate, and
in the feature amount selection candidate extraction step or the measurement element design step, a measurable suitable feature amount, for which the measurement element is capable of being designed and which predicts or discriminates the features of the sample, is selected from the feature amount candidates.

2. The measurable suitable feature amount selection method according to claim 1,
wherein, in the feature amount selection candidate extraction step, one or a plurality of the feature amounts are selected from the feature amount candidates, and the feature amount selection candidate is extracted,
in the measurement element design step, the measurement element is designed for the feature amount selection candidate,
in the measurement element design result notification step, at least one of the following is performed: in a case where it is determined that the design of the measurement element has failed on the basis of the result of the measurement element design step, the feature amount determined as a failure is fed back to the feature amount candidates and is deleted from the feature amount candidates; in a case where it is determined that the design of the measurement element has succeeded on the basis of the result of the measurement element design step, the feature amount determined as a success is used as the measurable suitable feature amount, the measurable suitable feature amount is fed back to the feature amount selection candidate extraction step, and conditions of the feature amount selection candidate extraction step are controlled; or the measurable suitable feature amount is fed back to the measurement element design step, and conditions of the measurement element design step are controlled, and
the feature amount selection candidate extraction step and the measurement element design step are repeated until a desired number of the measurable suitable feature amounts are capable of being selected.

3. The measurable suitable feature amount selection method according to claim 1,
wherein the measurement element design step is performed on the feature amount candidates, and a feature amount for which the design of the measurement element is determined to succeed is used as a measurable feature amount candidate,
in the measurement element design result notification step, a notification of the measurable feature amount candidate is sent to the feature amount selection candidate extraction step, and
in the feature amount selection candidate extraction step, the extraction is performed until a desired number of the measurable suitable feature amounts are capable of being selected from the measurable feature amount candidates.

4. The measurable suitable feature amount selection method according to claim 1,
wherein the feature amount selection candidate extraction step includes a first feature amount selection candidate extraction step of extracting the feature amount selection candidate with first feature amount selection means having a weak constraint and a second feature amount selection candidate extraction step of extracting the feature amount selection candidate with second feature amount selection means having a strong constraint,
the first feature amount selection candidate extraction step is performed on the feature amount candidates to extract first feature amount selection candidates,
the measurement element design step is performed on the first feature amount selection candidates, and a feature amount for which the design of the measurement element is determined to succeed is used as a measurable feature amount selection candidate,
in the measurement element design result notification step, a notification of the measurable feature amount selection candidate is sent to the second feature amount selection candidate extraction step, and
in the second feature amount selection candidate extraction step, the extraction is performed until a desired number of the measurable suitable feature amounts are capable of being selected from the measurable feature amount selection candidates.

5. The measurable suitable feature amount selection method according to claim 1,
wherein the measurement element design step includes a first measurement element design step of designing the measurement element for the feature amount with first measurement element design means having a weak constraint and a second measurement element design step of designing the measurement element for the feature amount with second measurement element design means having a strong constraint,
the first measurement element design step is performed on the feature amount candidates, and a feature amount for which the design of the measurement element is determined to succeed is used as a measurable feature amount candidate,
in the measurement element design result notification step, a notification of the measurable feature amount candidate is sent to the feature amount selection candidate extraction step, and the feature amount selection candidate extraction step is performed on the measurable feature amount candidates to extract a measurable feature amount selection candidate, and
the second measurement element design step is performed on the measurable feature amount selection candidates, a feature amount for which the design of the measurement element is determined to succeed is extracted as the measurable suitable feature amount, and the extraction is performed until a desired number of the measurable suitable feature amounts are capable of being selected.

6. The measurable suitable feature amount selection method according to claim 1, further comprising:
a repetition step of optionally repeating the feature amount selection candidate extraction step and the measurement element design step,
wherein, in the feature amount selection candidate extraction step, a feature amount selection candidate is selected from the feature amount candidates or the measurable feature amounts by one feature amount selection means selected from a plurality of feature amount selection means,
the measurement element design step is performed on the feature amount candidates or the feature amount selection candidates by one measurement element design means selected from a plurality of measurement element design means, and a feature amount for which the design of the measurement element is determined to succeed is selected as a measurable feature amount candidate,
in the measurement element design result notification step, a notification of the measurable feature amount candidate is sent to the feature amount selection candidate extraction step, and
the measurable suitable feature amount for which the measurement element is capable of being designed is selected in stages by the repetition step.

7. The measurable suitable feature amount selection method according to claim 6,
wherein the feature amount selection candidate extraction step is performed in ascending order of constraint in the repetition step, and
the measurement element design step is performed in ascending order of constraint in the repetition step.

8. The measurable suitable feature amount selection method according to any one of claims 1 to 7,
wherein the feature amount is information of a gene, and
the measurement element is gene measurement means.

9. The measurable suitable feature amount selection method according to claim 8,
wherein the information of the gene is information of a DNA methylation site, and
the gene measurement means is a primer.

10. The measurable suitable feature amount selection method according to any one of claims 1 to 9,
wherein a problem of predicting or discriminating the features of the sample is a multi-class classification problem of determining which of N classes the sample belongs to.

11. A measurable suitable feature amount selection program causing a computer to execute the measurable suitable feature amount selection method according to any one of claims 1 to 10.

12. A non-transitory computer-readable recording medium storing the program according to claim 11.

13. A measurable suitable feature amount selection device for selecting a feature amount that is used to predict or discriminate features of a sample and is measurable, the measurable suitable feature amount selection device comprising:
a processor,
wherein the processor is configured to have:
a data set input process of inputting a data set of a sample group in which values of a plurality of the feature amounts are recorded;
a feature amount candidate extraction process of extracting a feature amount candidate from the data set;
a feature amount selection candidate extraction process of selecting a feature amount for predicting or discriminating the features of the sample and extracting a feature amount selection candidate;
a measurement element design process of designing a measurement element that is capable of measuring the feature amount and extracting a feature amount, for which the design of the measurement element has succeeded, as a measurable feature amount; and
a measurement element design result notification process of feeding back a result of whether the design of the measurement element has succeeded or failed in the measurement element design process to the feature amount selection candidate extraction process or notifying the feature amount selection candidate extraction process of the measurable feature amount,
the feature amount selection candidate extraction process is performed on the feature amount candidate or the measurable feature amount, and the measurement element design process is performed on the feature amount candidate or the feature amount selection candidate, and
in the feature amount selection candidate extraction process or the measurement element design process, a measurable suitable feature amount, for which the measurement element is capable of being designed and which predicts or discriminates the features of the sample, is selected from the feature amount candidates.

14. The measurable suitable feature amount selection device according to claim 13,
wherein, in the feature amount selection candidate extraction process, one or a plurality of the feature amounts are selected from the feature amount candidates, and the feature amount selection candidate is extracted,
in the measurement element design process, the measurement element is designed for the feature amount selection candidate,
in the measurement element design result notification process, at least one of the following is performed: in a case where it is determined that the design of the measurement element has failed on the basis of the result of the measurement element design process, the feature amount determined as a failure is fed back to the feature amount candidates and is deleted from the feature amount candidates; in a case where it is determined that the design of the measurement element has succeeded on the basis of the result of the measurement element design process, the feature amount determined as a success is used as the measurable suitable feature amount, the measurable suitable feature amount is fed back to the feature amount selection candidate extraction process, and conditions of the feature amount selection candidate extraction process are controlled; or the measurable suitable feature amount is fed back to the measurement element design process, and conditions of the measurement element design process are controlled, and
the feature amount selection candidate extraction process and the measurement element design process are repeated until a desired number of the measurable suitable feature amounts are capable of being selected.

15. The measurable suitable feature amount selection device according to claim 13,
wherein the measurement element design process is performed on the feature amount candidates, and a feature amount for which the design of the measurement element is determined to succeed is used as a measurable feature amount candidate,
in the measurement element design result notification process, a notification of the measurable feature amount candidate is sent to the feature amount selection candidate extraction process, and
in the feature amount selection candidate extraction process, the extraction is performed until a desired number of the measurable suitable feature amounts are capable of being selected from the measurable feature amount candidates.
